# FASCICULE DE BREVET EUROPEEN

(11) **EP 2 040 754 B1**
(45) Date de publication et mention de la délivrance du brevet: **25.01.2012**
(21) Numéro de dépôt: 07765378.0
(22) Date de dépôt: 12.06.2007
(51) Int. Cl.: A61K 45/00, A61P 17/02, A61P 43/00

(54) **UTILISATION DE FRACTIONS CELLULAIRES DU TISSU ADIPEUX POUR LA REGENERATION TISSULAIRE POST IRRADIATION**
VERWENDUNG VON FETTGEWEBEZELLFRAKTIONEN ZUR GEWEBEREGENERATION NACH EINER BESTRAHLUNG
USE OF ADIPOSE-TISSUE CELL FRACTIONS FOR POST-IRRADIATION TISSUE REGENERATION

(30) Priorité: 12.06.2006 FR 0605190
(43) Date de publication de la demande: 01.04.2009
(73) Titulaire: Institut de Radioprotection et de Sûreté Nucléaire, 92260 Fontenay aux Roses (FR)
(72) Inventeur: TAMARAT, Radia, 92330 SCEAUX (FR); BENDERITTER, Marc, 94240 L'hay Les Roses (FR)
(74) Mandataire: Ahner, Francis
(86) Numéro de dépôt international: PCT/EP2007/055782
(87) Numéro de publication internationale: WO 2007/144358

(56) Documents cités:
- WO-A2-01/62901
- FR-A- 2 859 381
- FR-A1- 2 819 265
- US-A1- 2003 082 152
- VRIES DE H J C ET AL: "STROMAL CELLS FROM SUBCUTANEOUS ADIPOSE TISSUE SEEDED IN A NATIVE COLLAGEN/ELASTIN DERMAL SUBSTITUTE REDUCE WOUND CONTRACTION IN FULLTHICKNESS SKIN DEFECTS" LABORATORY INVESTIGATION, UNITED STATES AND CANADIAN ACADEMY OF PATHOLOGY, BALTIMORE,, US, vol. 73, no. 4, 1995, pages 532-540, XP001041841 ISSN: 0023-6837
- GIMBLE J M: "ADIPOSE TISSUE-DERIVED THERAPEUTICS" EXPERT OPINION ON BIOLOGICAL THERAPY, ASHLEY, LONDON, GB, vol. 3, no. 5, août 2003 (2003-08), pages 705-713, XP009044158 ISSN: 1471-2598
- CASTEILLA L ET AL: "Adipose tissue, plastic and reconstructive surgery: come back to sources" ANNALES DE CHIRURGIE PLASTIQUE ESTHETIQUE, EXPANSION SCIENTIFIQUE FRANCAISE, PARIS, FR, vol. 49, no. 5, octobre 2004 (2004-10), pages 409-418, XP004615058 ISSN: 0294-1260

## Description

La présente invention concerne l'utilisation de cellules issues de la fraction du stroma vasculaire du tissu adipeux extra médullaire, pour favoriser la régénération de la peau à la suite de lésions causées par irradiation.

Le processus cicatriciel nécessite la mise en oeuvre de mécanismes biologiques et moléculaires complexes et parfaitement orchestrés, tels que la migration cellulaire, la prolifération cellulaire, ou le dépôt de matrice extracellulaire. Une lésion induite constitue le point de départ d'une cascade d'événements comprenant des interactions entre des facteurs de croissances locaux, régionaux et systémiques, ainsi que la participation au niveau cellulaire de différents acteurs dont les cellules souches de la moelle osseuse. Une perturbation dans ces mécanismes complexes a des conséquences invalidantes pour les sujets atteints, notamment des conséquences physiques ou sur leur qualité de vie et peuvent même aller jusqu'à la mort. C'est le cas lorsque des infections apparaissent et conduisent dans les cas les plus graves à des amputations de membres, ou plus généralement à des interventions chirurgicales lourdes.

Dans le domaine des lésions cutanées, une blessure provoque une destruction plus ou moins profonde des tissus, et une hypoxie qui conduit à une régulation de facteurs de croissance, une activation de la dégradation de la matrice, et une stimulation de l'angiogenèse. Après seulement quelques heures, la migration et la prolifération de cellules épidermales et dermales constituent les facteurs déclenchant de la re-épithélialisation. La formation d'un nouveau réseau vasculaire est également nécessaire pour soutenir les tissus nouvellement formés. Toutefois, il a été démontré que de nombreuses situations pathologiques conduisent à des dysfonctionnements dans l'angiogenèse, à la perte de kératinocytes, et détériorent ainsi la régénération de la peau.

Les thérapies actuellement utilisées font essentiellement appel à la chirurgie, accompagnées parfois de l'administration de médicaments adaptés tels que des antibiotiques. Des thérapies faisant appel à des facteurs de croissances ont aussi été envisagées, notamment avec l'administration de PDGF-BB et de bFGF, ou encore le recours à des thérapies cellulaires. Elles permettent de favoriser la réparation des plaies et peuvent être utilisées seules ou en complément d'autres thérapies ou chirurgies. Des techniques mettant en oeuvre des cellules régénératrices ont connu un relatif succès. Elles consistent à utiliser l'habilité des cellules souches à s'auto-renouveler indéfiniment, et à se différentier en cellules et tissus matures spécialisés. Toutefois ces méthodes requièrent des technologies de pointe et engendrent des coûts très important dus à la disponibilité des matières premières, à leur obtention et à leur purification. Elles sont également très dépendantes d'éventuels phénomènes de contamination par des micro-organismes et de délais relativement importants pour la préparation des matières premières. Ces thérapies cellulaires semblent être les plus prometteuses.

Ainsi, la demande US 2003/0082152 (1) décrit de façon assez générale des procédés de préparation de cellules souches dérivées du tissu adipeux, ainsi que leur caractère pluripotent et l'intérêt de ces cellules pour l'ingénierie tissulaire, la réparation des blessures, et la régénération tissulaire in vivo et ex vivo. Les types de blessures pour lesquelles ces cellules peuvent être utilisées pour favoriser la cicatrisation ne comprennent que des blessures d'origine mécanique. Ce document n'indique à aucun endroit que des lésions induites par irradiation peuvent également être traitées.

De même, De Vries et al. 1995 (2) décrit le test de substituts dermiques ensemencés avec différents types de populations cellulaires sur la cicatrisation de lésions cutanées induites de façon mécanique. Les populations cellulaires testées incluent des fibroblastes autologues, une population appelée fraction du stroma vasculaire (SVF) dérivée du tissu adipeux, ainsi que cette même fraction stromale vasculaire dans laquelle les fragments vasculaires ont été ôtés (SF). Les résultats obtenus dans cet article montrent que les substituts dermiques ensemencés avec la population SF, de même que les substituts ensemencés avec des fibroblastes autologues, permettent d'améliorer la cicatrisation de la blessure cutanée induite de façon mécanique. Ici aussi, il n'est ni décrit ni suggéré que des lésions induites par irradiations, beaucoup plus complexes, pourraient être traitées de la même façon.

Comme indiqué précédemment, différents états pathologiques perturbent les phénomènes de réparation des lésions. C'est le cas des irradiations qui modifient l'angiogenèse et la re-épithélialisation. Plusieurs études ont ainsi montré que des radiations pouvaient conduire à une desquamation sèche associée à une kératinisation atypique de la peau, ou bien à une perte d'épiderme accompagnée d'ulcération. Des changements vasculaires se produisent également, incluant des occlusions, des oedèmes, des thromboses, ou des pertes de vaisseaux. Par exemple, au niveau cutané, les lésions sont de natures différentes de celles que l'on observe dans le cas de blessures ou autres lésions pathologiques. Ces phénomènes se traduisent par un ralentissement du processus de cicatrisation.

En particulier, l'irradiation locale cutanée affecte très fortement le processus cicatriciel. Des lésions précoces, considérées comme provenant de l'atteinte de la couche basale proliférante de l'épiderme, et tardives, reliées aux atteintes du derme et des tissus sous-jacents vont se manifester après exposition à de fortes doses. Le premier symptôme observable est la radiodermite, définie comme une réaction inflammatoire de la peau, équivalente à une brûlure du premier degré, puis la dépilation. La dilatation des capillaires provoque également un érythème aigu. Enfin, une desquamation sèche puis humide peut apparaître quelques jours après une irradiation de 10 à 20 Gray et se caractérise d'une part, par une dégénérescence des kératinocytes au niveau de l'épiderme conduisant à son amincissement à l'aplatissement des papilles dermiques et d'autre part, par le gonflement et le prolifération de l'endothélium vasculaire. Les effets tardifs se traduisent par une ischémie provenant probablement de l'hyperprolifération des cellules épithéliales survivantes provoquant l'occlusion localisée ou partielle des artérioles et par une fibrose majeure. Ces phénomènes physiologiques ne sont pas présents dans les lésions cutanées de type blessure infligée de façon mécanique, qui sont donc d'un type différent de celles causées par irradiation.

Notamment, le fait qu'un médicament soit efficace pour améliorer la cicatrisation d'une blessure induite de façon mécanique n'implique pas nécessairement que ce médicament sera efficace pour le traitement des lésions, notamment cutanées, liées à une irradiation, la guérison d'une lésion post-irradiation faisant intervenir des mécanismes beaucoup plus complexes.

Dans ce contexte post-irradiation, qu'il soit accidentel, ou provoqué par une radiothérapie, il est donc très important de pouvoir mettre en oeuvre des mécanismes efficaces de réparation des lésions, notamment cutanées.

Pour la mise en oeuvre de thérapies cellulaires, différentes sources de cellules souches ont été envisagées. Dans un premier temps les cellules souches ont été isolées à partir de tissus embryonnaires, mais récemment la présence de cellules souches pluripotentes a été mise en évidence dans différents tissus adultes tels que la moelle osseuse, la peau, le cerveau, les muscles, et le tissu adipeux. De telles cellules souches sont ainsi plus facilement disponibles que des cellules embryonnaires, et leur utilisation ne comporte pas les mêmes problèmes éthiques. L'utilisation de cellules souches est limitée par leur faible nombre dans la plupart des tissus adultes, et par la difficulté de les extraire et de les purifier. De nouvelles voies d'accès ont été proposées (3) pour l'obtention de cellules souches, en particulier à partir du tissu adipeux.

Le tissu adipeux peut en effet constituer un réservoir important, dans lequel les cellules souches sont comparativement faciles à isoler, et en des quantités relativement importantes. Elles permettent en outre de s'affranchir de l'existence d'un donneur compatible comme c'est le cas lors de l'utilisation de cellules provenant de la moelle osseuse. Elles permettent aussi d'envisager des « auto-greffes », évitant ainsi les échecs liés à d'éventuels rejets.

Le tissu adipeux existe sous différentes formes chez les mammifères. Le tissu adipeux blanc extramedullaire, principal organe de réserve de l'organisme, le tissu adipeux blanc medullaire, et le tissu adipeux brun thermogénique. Le tissu adipeux blanc constitue une source abondante de cellules, faciles à obtenir. De plus son potentiel persiste tout au long de la vie. Il est constitué de deux fractions cellulaires : une fraction adipocytaire caractérisée par l'accumulation de triglycérides, et composée en grande majorité d'adipocytes différentiés, et une fraction non-adipocytaire appelée fraction du stroma vasculaire (FSV) comprenant des cellules sanguines, des cellules endothéliales matures, des péricytes, des fibroblastes, et des cellules souches pluripotentes. La fraction du stroma vasculaire comprend, outre des progéniteurs adipocytaires, des progéniteurs hématopoïétiques et neurogéniques, ainsi que des cellules souches mésenchymateuses capables de se différentier en lignées oestrogéniques, chondrogéniques, et myogéniques (4).

Le tissu adipeux blanc possède des propriétés angiogéniques ayant des applications en thérapie cellulaire autologue dans un contexte post-traumatique ou pathologique. L'injection de tissus adipeux autologue est notamment pratiquée en chirurgie pour favoriser la re-vascularisation des greffes et la reconstruction des tissus mous. Des cellules issues de la FSV ont aussi été administrées pour favoriser l'angiogenèse dans le traitement de pathologies ischémiques. Les cellules de la FSV représentent une population hétérogène de cellules entourant les adipocytes dans les tissus gras, et incluent des cellules endothéliales microvasculaires matures. Cette fraction a aussi été identifiée comme source importante de cellules pluripotentes capables de se différencier en phénotypes neurogéniques, cardiomyocytes, et plus récemment comme possédant une activité angiogénique. La FSV intervient dans la formation d'un modèle de matrigel et augmente la néovascularisation de tissus ischémiés (5). De plus, des lignées cellulaires adipocytaires possèdent la capacité de libérer des facteurs pro-angiogéniques importants tels que le monobutyril, le facteur de croissance vasculaire endothélial (VEGF) et la leptine.

L'utilisation de cellules issues du FSV dans les thérapies cellulaires présente donc un intérêt majeur et reconnu. Ainsi elles ont été proposées dans le cadre du traitement des myopathies, des cardiopathies, et d'autres maladies dans lesquelles on observe une dégénérescence musculaire. Leur intérêt dans la reconstruction de réseaux vasculaires fonctionnels a aussi été étudié.

Les cellules souches issues de fractions cellulaires du stroma adipeux sont également utilisées dans l'ingénierie tissulaire, notamment dans la régénération du cartilage humain, dans les greffes vasculaires et dans la cicatrisation de plaies (6).

La Demanderesse a, de façon surprenante, mis en évidence que des cellules issues de la fraction du stroma vasculaire du tissu adipeux extra médullaire présentent un intérêt considérable dans le traitement de lésions dans un contexte précis de post-irradiations.

Ainsi la présente invention concerne l'utilisation de cellules issues de la fraction du stroma vasculaire du tissu adipeux extra médullaire, pour la préparation d'un médicament destiné à favoriser la régénération de la peau à la suite de lésions causées par irradiation.

Dans un aspect avantageux, l'invention concerne l'utilisation de cellules issues de la fraction du stroma vasculaire du tissu adipeux extra médullaire, pour la préparation d'un médicament destiné à favoriser la régénération des kératinocytes à la suite de lésions causées par des irradiations. Plus particulièrement les lésions sont des plaies cutanées causées par irradiations.

Jusqu'alors, de nombreux travaux ont proposé de stimuler le processus de cicatrisation de manière pharmacologique, par l'administration d'agents pro-angiogéniques, ou de molécules pro-inflammatoires. Les méthodes cellulaires connues font appel à l'utilisation de cellules de la moelle osseuse, ou à des cellules du sang. Dans le contexte de cicatrisation pathologique post-irradiation, des cellules issues du tissu adipeux n'ont jamais été utilisées.

Les lésions et plus particulièrement les plaies et/ou des brûlures cutanées causées par des irradiations, qu'elles soient accidentelles ou provoquées par des traitements de radiothérapie, sont de nature différentes des traumatismes physiopathologiques. L'épiderme et les kératinocytes sont atteints, l'élasticité des tissus est diminuée et le processus de cicatrisation est considérablement ralenti.

L'utilisation de cellules issues de la FSV du tissu adipeux extra médullaire permet d'agir en accélérant la fermeture des cicatrices, en agissant au niveau de la différentiation des kératinocytes, et/ou de la viscoélasticité de la peau en particulier par augmentation de la production de collagène. Elle présente l'avantage de pouvoir disposer d'une quantité de tissus et de cellules très importante et ne pose pas de problèmes éthiques particuliers. Il est en outre possible d'envisager des greffes homologues ou hétérologues, et la capacité à traiter plusieurs personnes à partir d'un seul individu. La mise en oeuvre de l'utilisation selon l'invention est grandement facilitée par la provenance des cellules utilisées. En effet il est possible de maintenir, multiplier, voire d'immortaliser les cellules in vitro dans un milieu défini. Il est envisageable de maintenir les cellules congelées et de constituer des stocks de cellules disponibles.

Les lésions et plus particulièrement les plaies cutanées étant réparées beaucoup plus rapidement lors de l'utilisation selon la présente invention, les tissus sont plus rapidement protégés de tout agent pathogène extérieur.

Les avantages en terme de coût sont également importants. L'obtention des cellules souches est facilitée par leur provenance, et les protocoles sont plus simples et moins coûteux. En effet beaucoup de prélèvements effectués notamment lors de liposuccion ou de dermolipectomie sont d'habitude détruits. Les méthodes de prélèvement sont peu invasives, et les temps d'hospitalisation et de soins qui en découlent pour les patients s'en trouvent considérablement réduits. Tous ces facteurs contribuent à diminuer le coût de la mise en oeuvre des traitements évoqués ci-dessus.

L'utilisation de cellules issues de la fraction du stroma vasculaire du tissu adipeux extra médullaire pour favoriser la régénération tissulaire à la suite de lésions causées par irradiation, dans des tissus choisis parmi les os, le cerveau, le cou, l'intestin, les seins, le coeur, les poumons, l'utérus et le rectum est aussi décrite ici.

L'utilisation de cellules issues de la fraction du stroma vasculaire du tissu adipeux extra médullaire destiné à favoriser la régénération de la peau à la suite de lésions causées par irradiation selon la présente invention trouve une application dans de nombreux domaines. Les radiations responsables des lésions peuvent être accidentelles, ou bien causées par des traitements de radiothérapie. C'est en particulier le cas pour les patients atteints de cancer.

Les médicaments utilisés dans le cadre de la présente invention peuvent être utilisés à titre préventif et administrés avant l'irradiation. Ils peuvent bien sûr aussi être utilisés à titre curatif, et administrés après l'irradiation.

Les cellules du tissu adipeux sont directement obtenues à partir de fragments de tissus adipeux prélevés sous anesthésie. Après digestion enzymatique de la matrice extra-cellulaire, les différentes populations cellulaires sont sélectionnées par différence de densité ou par différence d'expression d'antigènes. La fraction du stroma-vasculaire utilisée pour la mise en oeuvre de la présente invention ne contenant pas les adipocytes est ainsi isolée.

Selon un mode de réalisation avantageux de l'invention, les cellules sont obtenues par les étapes successives suivantes :
- prélèvement d'un échantillon de tissu adipeux extra-médullaire,
- isolement selon les procédés décrits par Bjomtorp et al (7) de la fraction cellulaire du stroma vasculaire par digestion de la matrice par des enzymes protéolytiques, et par séparation selon le gradient de densité,
- purification des cellules par séparation physique et/ou par immunosélection.

La séparation physique est réalisée par différence d'adhésion sur un support solide, et l'immunosélection est réalisée à l'aide de plusieurs anticorps spécifiques des marqueurs exprimés par les précurseurs adipocytaires (CD 34+,CD45- et CD31-).

Les cellules issues de la FSV du tissu adipeux extra médullaire sont utilisées selon l'invention pour préparer un médicament se présentant sous la forme d'une composition pharmaceutique contenant les cellules issues de la fraction du stroma vasculaire du tissu adipeux extra médullaire, et au moins un véhicule et/ou support pharmaceutiquement acceptable. Cette composition pharmaceutique est adaptée à toute forme d'administration habituellement envisagée. Parmi les formes pharmaceutiques, on peut citer plus particulièrement celles qui conviennent à l'administration parentérale, per- ou transcutanée, les crèmes, les pommades, les sprays, les gels, les ampoules et/ou solutés injectables. La posologie varie avec l'âge et le poids du patient, la voie d'administration, ou les éventuels traitements associés. Dans un aspect de l'invention, l'utilisation des cellules issues de la fraction du stroma vasculaire du tissu adipeux extra médullaire, est destinée à la préparation d'un médicament sous forme de spray, de gel, ou de soluté injectable. En particulier ces compositions pharmaceutiques peuvent se présenter sous forme d'aérosols.

Dans un autre aspect de l'invention les cellules issues de la fraction du stroma vasculaire du tissu adipeux extra médullaire sont utilisées pour la préparation de dermes équivalents, tel que l'INTEGRA^{®}, et destinés à favoriser la régénération tissulaire à la suite de lésions causées par irradiation. Ces dermes équivalents sont destinés à administrer les cellules dans une matrice de collagène que constitue l'INTEGRA^{®}). Le support polymérique ou copolymérique est solide ou semi-solide

L'INTEGRA^{®} est une peau artificielle, ou structure de régénération cutanée. C'est un système de membrane à double couche visant à un remplacement cutané. La couche de remplacement dermique est fabriquée notamment à partir d'une matrice poreuse de fibres de collagène de tendon bovin réticulées et d'un glyscosaminoglycane dont la porosité est contrôlée et la vitesse de dégradation définie lors de sa fabrication.

Plus particulièrement, dans cet aspect, les Dermes équivalents sont utile dans le traitement des brûlures causées par irradiation.

Les exemples suivants illustrent l'invention mais ne la limitent en aucune façon.

### Description des figures :

- Figure 1 :: Observation de l'évolution de la cicatrice des souris dans un contexte pathologique, post-irradiation.
- Figure 2 :: Observation de l'évolution de la cicatrice des souris dans un contexte physiologique, sans irradiation.
- Figure 3 :: Histogrammes des mesures de l'ouverture de cicatrice restante à J 14 (Fig. 3A), et de la viscoélasticité à J 14 (Fig. 3B).

### Exemple 1 : Obtention et préparation des tissus adipeux.

Un fragment de tissu adipeux est prélevé au niveau du tissu adipeux sous cutané chez la souris sous anesthésie. Après digestion de la matrice extra-cellulaire par des enzymes protéolytiques, à 37° C dans de la collagénase pendant 45 min, pour permettre la dissociation des cellules du tissu, les différentes populations cellulaires sont sélectionnées par différence de densité selon les procédé décrits par Bjomtorp et al (6) ou par différence d'expression d'antigènes. La fraction cellulaire qui ne contient pas les adipocytes correspond à la fraction du stroma vasculaire. Ces cellules sont marquées CD 34 +, CD 45- et CD 31.

### Exemple 2 : Effet des fractions cellulaires selon l'invention sur la cicatrisation.

Une irradiation localisée de 20 Gy est réalisée sur la face dorsale de l'animal après avoir rasé et anesthésié les souris. Cette irradiation est réalisée avant l'application d'un punch.

### ■ Modèle de cicatrisation :

On applique un punch de 0,8 mm de diamètre sur la face dorsale de souris mâles âgées de 8 semaines (C57B16, Iffa Creddo), afin de réaliser une plaie. Immédiatement après l'application du punch, les cellules obtenues à l'exemple 1 sont injectées en local (loc) ou par voie intra-veineuse (iv). Les animaux reçoivent 1x10⁶ cellules et les contrôles reçoivent le solvant, du tampon phosphate salin.

Chaque groupe d'expérience est constitué de 5 individus.

Les expériences sont répétées sans irradiation préalable des souris.

### ■ Evaluation de la technique de cicatrisation :

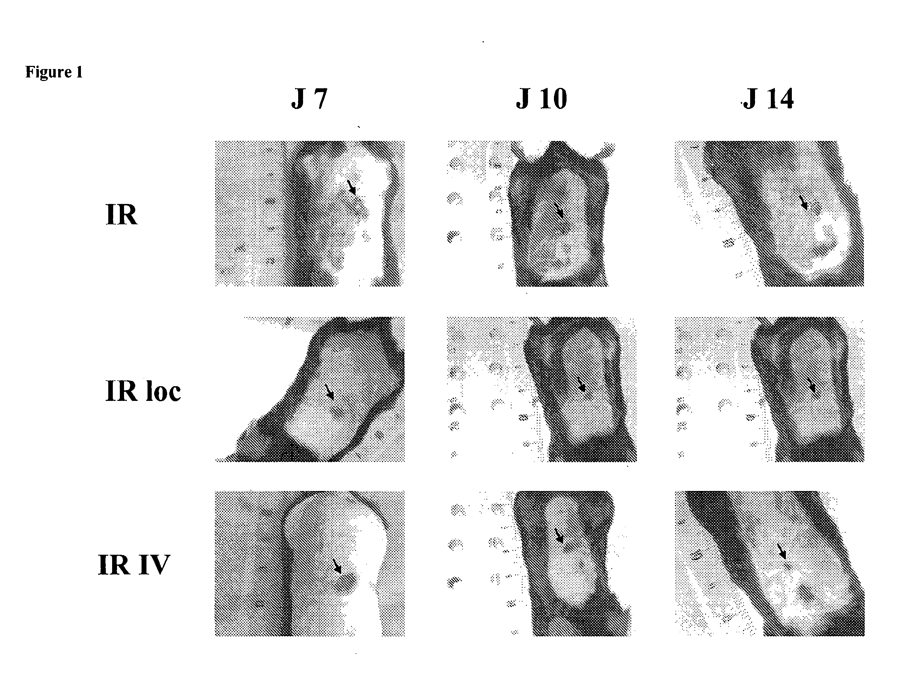

La diminution de la surface de la plaie est évaluée par observation directe à différents temps : J0, J4, J7, J10 et J14. L'évolution de la cicatrice des souris est suivie par photographie. Les résultats sont reportés à la figure 1 (IR, IR Loc, IR iv).

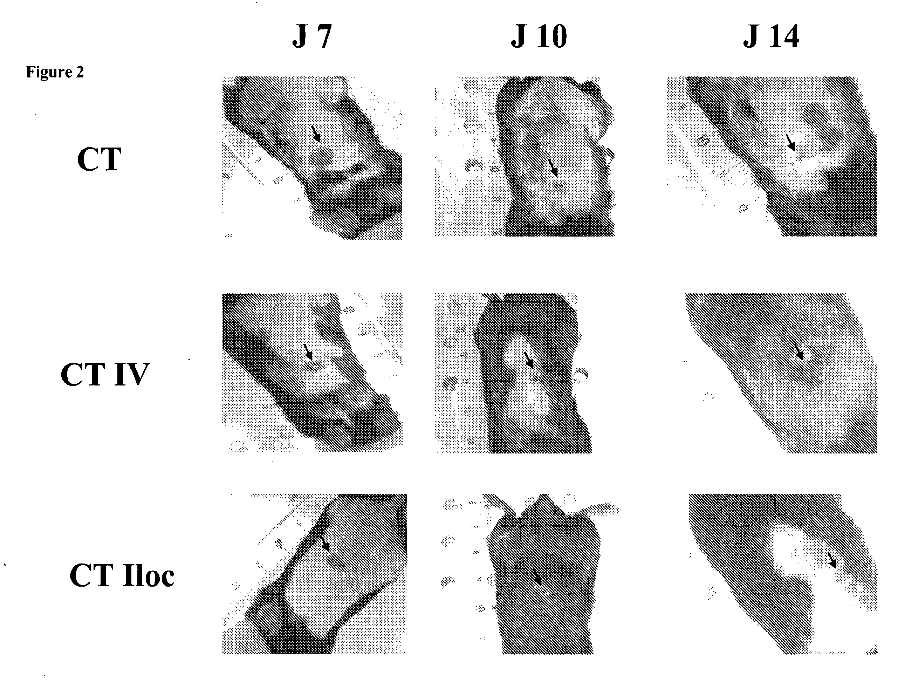

Ces observations sont comparées à l'évolution de la même cicatrice réalisée dans un contexte physiologique (CT, CT Loc, CT iv), c'est-à-dire sans irradiation préalable (voir figure 2).

Les mesures de surfaces sont réalisées à l'aide d'un logiciel d'analyse d'image (Histolab), dans les deux conditions de cicatrisations : physiologique et pathologique. Elles sont reportées dans le tableau (I) suivant qui donne les valeurs de l'ouverture de cicatrice restant à J14. Ces valeurs sont exprimées en pourcentage restant par rapport à J0 fixé comme étant 100 %.

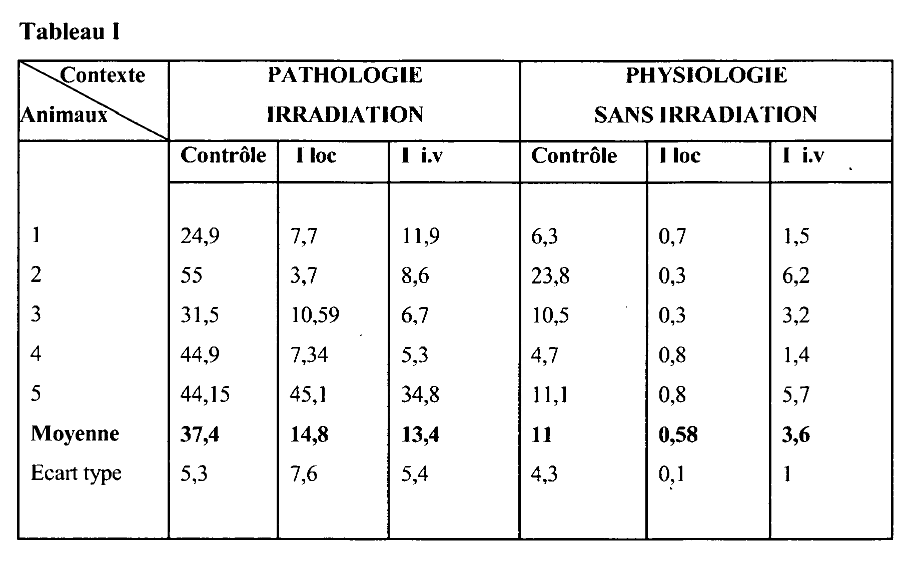

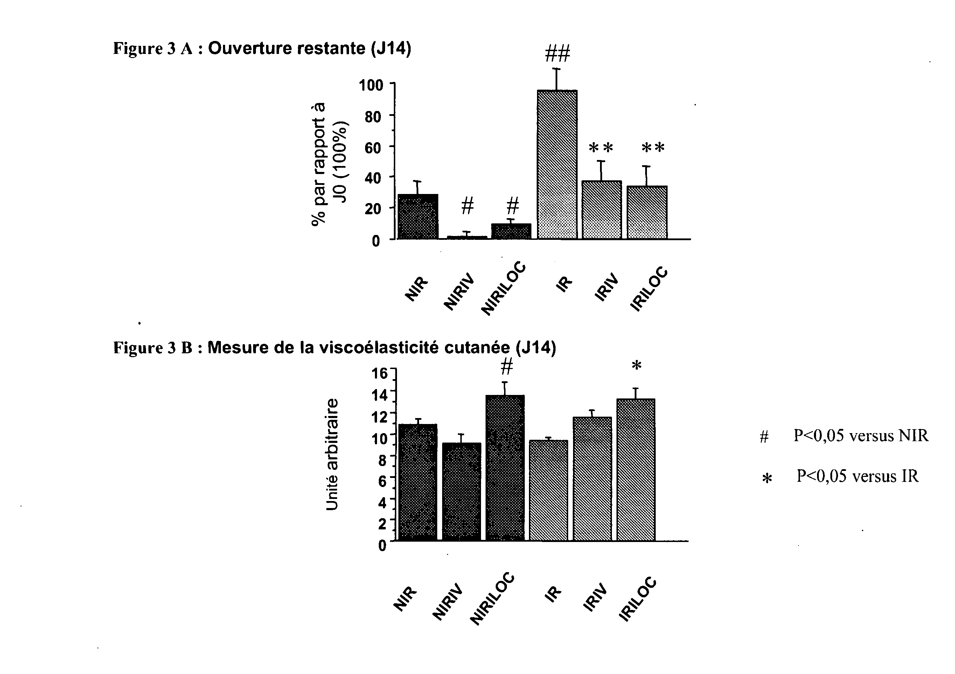

Les histogrammes correspondant sont représentés figure 3A.

### ■ Mesure de la qualité de la cicatrice par la technique du cutomêtre :

Cette mesure à pour but de vérifier que la régénération tissulaire est associée à une amélioration de la qualité du tissu. En fin de processus cicatriciel, une sonde de 2 mm est appliquée pour aspirer la peau dorsale de l'animal et estimer le paramètre de viscoélasticité cutané au niveau du tissu régénéré. L'application de la sonde permet de réaliser une succion puis une relaxation de la peau. La déformation de la peau est estimée à l'aide d'un système de mesure optique qui permet de délivrer un graphique avec des mesures de l'élasticité et de la relaxation cutané. De la même façon que précédemment, les mesures sont réalisées en parallèle dans un contexte pathologique, sur des animaux ayant été irradiés, et dans un contexte physiologique, sur des animaux n'ayant pas subit d'irradiation préalable.

Les résultats des mesures de viscoélasticité sont reportés dans le tableau (II) ci-dessous, exprimés en unités arbitraires.

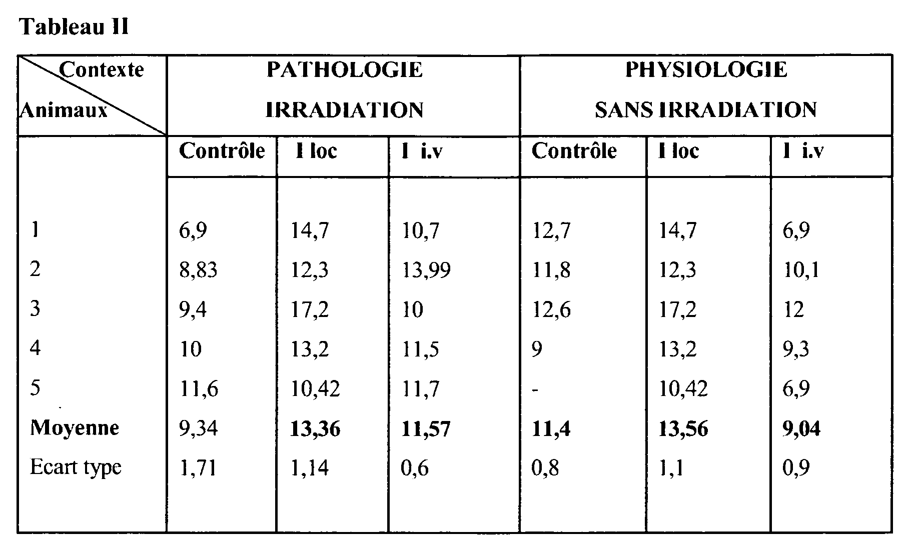

Les histogrammes correspondant sont représentés sur la figure 3B.

### ■ Conclusion :

En l'absence de cellules de la FSV du tissu adipeux extra-médullaire, la réparation tissulaire dans un contexte post-irradiation se fait de manière tardive, et la reconstitution n'est que partielle. En effet en fin de processus, (J14) la surface d'ouverture de la plaie reste importante.

Les souris ayant reçu les cellules de la FSV du tissu adipeux extra-médullaire ont complètement reconstitué leur peau avec une fermeture complète de la plaie et une amélioration de la qualité de la peau, et plus précisément de sa viscoélasticité.

Le ralentissement observé lors de la fermeture de plaies causées dans un contexte post-irradiation est totalement supprimé par l'utilisation des cellules de la FSV du tissu adipeux extra-médullaire selon l'invention, et les valeurs observées montrent que l'accélération du processus de cicatrisation obtenue après l'administration des ces cellules dans un contexte d'irradiation permet d'obtenir une plaie comparable à se que l'on observe dans une situation de cicatrisation physiologique normale.

### REFERENCES BIBLIOGRAPHIQUES

1. US 2003/0082152
2. De Vries et al. Lab Invest. 1995 Oct, 73(4), p.532-40
3. WO-2005/035742
4. WO-02/055678
5. WO 2005/025584
6. Gimble et al. Exp. Opin. Biol. Therap., 2003, 3(5), p.705-713
7. Bjomtorp et al. J.Lipid Res., 1978, 19, p. 316-324

## Revendications

1. Utilisation de cellules issues de la fraction du stroma vasculaire du tissu adipeux extra médullaire, pour la préparation d'un médicament destiné à favoriser la régénération de la peau à la suite de lésions causées par irradiation.

2. Utilisation selon la revendication 1, **caractérisée en ce que** le médicament est destiné à favoriser la régénération des kératinocytes.

3. Utilisation selon l'une des revendications 1 à 2, **caractérisée en ce que** les lésions sont des plaies cutanées causées par irradiation.

4. Utilisation selon l'une des revendications 1 à 3, **caractérisée en ce que** les lésions sont causées par irradiation consécutive à une radiothérapie.

5. Utilisation selon l'une des revendications 1 à 4, **caractérisée en ce que** les lésions sont causées par irradiation accidentelle.

6. Utilisation selon l'une des revendications 1 à 5, **caractérisée en ce que** le médicament est administré à titre préventif, avant l'irradiation.

7. Utilisation selon l'une des revendications 1 à 5, **caractérisée en ce que** le médicament est administré à titre curatif, après l'irradiation.

8. Utilisation selon la revendication 4, **caractérisée en ce que** le médicament est destiné à être administré à des patients atteints de cancer.

9. Utilisation selon l'une des revendications 1 à 8, **caractérisée en ce que** le médicament se présente sous la forme d'une crème, d'une pommade, d'un spray, d'un gel, d'ampoules et/ou de solutés injectables.

10. Utilisation selon l'une des revendications 1 à 8 **caractérisée en ce que** le médicament se présente sous la forme de derme équivalent.

11. Utilisation selon la revendication 10, caractérisée en que le derme équivalent est destiné au traitement des brûlures causées par irradiation.

## Claims

1. Use of cells derived from the cellular fraction of the vascular stroma of extramedullary adipose tissue for the preparation of a drug aimed at promoting the regeneration of skin in lesions caused by irradiation.

2. Use according to claim 1, wherein the drug is aimed at promoting the regeneration of keratinocytes.

3. Use according to claim 1 or claim 2, wherein the lesions are cutaneous wounds caused by irradiation.

4. Use according to one of claims 1 to 3, wherein the lesions are caused by irradiation as a result of radiotherapy.

5. Use according to one of claims 1 to 4, wherein the lesions are caused by accidental irradiation.

6. Use according to one of claims 1 to 5, wherein the drug is administered as a preventive measure, prior to irradiation.

7. Use according to one of claims 1 to 5, wherein the drug is administered as a curative measure, after irradiation.

8. Use according to claim 4, wherein the drug is intended to be administrated in cancer patients.

9. Use according to one of claims 1 to 8, wherein the drug is in the form of a cream, ointment, spray, gel, vial and/or injectable solutions.

10. Use according to one of claims 1 to 8, wherein the drug is in the form of equivalent dermis.

11. Use according to claim 10, wherein the equivalent dermis is intended for the treatment of burns caused by irradiation.

## Patentansprüche

1. Verwendung von Zellen, die aus einer stromal-vaskulären Fraktion von extramedullärem Fettgewebe stammen, zur Herstellung eines Medikaments zur Förderung der Regeneration der Haut nach Läsionen, die durch Strahlenbelastung verursacht wurden.

2. Verwendung gemäß Anspruch 1, **dadurch gekennzeichnet, dass** das Medikament zur Förderung der Regeneration von Keratinozyten bestimmt ist.

3. Verwendung gemäß einem der Ansprüche 1 bis 2, **dadurch gekennzeichnet, dass** die Läsionen kutane Wunden sind, die durch Strahlenbelastung verursacht wurden.

4. Verwendung gemäß einem der Ansprüche 1 bis 3, **dadurch gekennzeichnet, dass** die Läsionen durch Strahlenbelastung infolge einer Strahlentherapie hervorgerufen wurden.

5. Verwendung gemäß einem der Ansprüche 1 bis 4, **dadurch gekennzeichnet, dass** die Läsionen durch zufällige Strahlenbelastung verursacht wurden.

6. Verwendung gemäß einem der Ansprüche 1 bis 5, **dadurch gekennzeichnet, dass** das Medikament präventiv vor der Strahlenbelastung verabreicht wird.

7. Verwendung gemäß einem der Ansprüche 1 bis 5, **dadurch gekennzeichnet, dass** das Medikament kurativ nach der Strahlenbelastung verabreicht wird.

8. Verwendung gemäß Anspruch 4, **dadurch gekennzeichnet, dass** das Medikament an Patienten verabreicht wird, die an Krebs leiden.

9. Verwendung gemäß einem der Ansprüche 1 bis 8, **dadurch gekennzeichnet, dass** das Medikament in Form einer Creme, einer Salbe, eines Sprays, eines Gels, einer Ampulle und/oder von injizierbaren gelösten Stoffen vorliegt.

10. Verwendung gemäß einem der Ansprüche 1 bis 8, **dadurch gekennzeichnet, dass** das Medikament in Form eines Lederhautersatzes vorliegt.

11. Verwendung gemäß Anspruch 10, **dadurch gekennzeichnet, dass** der Lederhautersatz zur Behandlung von Verbrennungen bestimmt ist, die durch Strahlenbelastung verursacht wurden.
